Europäisches Patentamt

(19) European Patent Office

Office européen des brevets

(11) Publication number: **0 273 579 B1**

# (12) EUROPEAN PATENT SPECIFICATION

(45) Date of publication of patent specification: **08.01.92**    (51) Int. Cl.5: **A61K 7/28, A61K 37/50**

(21) Application number: **87310331.1**

(22) Date of filing: **23.11.87**

(54) A composition for periodontal use.

(30) Priority: **22.11.86 JP 279497/86**

(43) Date of publication of application:
**06.07.88 Bulletin 88/27**

(45) Publication of the grant of the patent:
**08.01.92 Bulletin 92/02**

(84) Designated Contracting States:
**AT BE CH DE FR GB IT LI LU NL SE**

(56) References cited:
**EP-A- 0 045 222**
**EP-A- 0 070 656**
**EP-A- 0 172 577**
**WO-A-87/01387**

**PATENT ABSTRACTS OF JAPAN, vol. 5, no. 85 (C-57) (757), 3rd June 1981; & JP-A-56 32422 (Mochida) 01-04-1981**

**PATENT ABSTRACTS OF JAPAN, vol. 5, no. 54 (C-50)(726), 15th April 1981; & JP-A-56 7720 (Mochida), 27-01-81**

**La Recherche, vol. 10, no. 106, December 1979, pages 1269-1270, Paris, FR; A.M. Michelson: Une enzyme qui nous veut du bien"**

**Chambers Dictionary, p. 1080**

(73) Proprietor: **Nakano, Minoru**
**7-9 Otemachi-3-chome**
**Maebashi-shi Gunma-ken(JP)**

(72) Inventor: **Nakano, Minoru**
**7-9 Otemachi-3-chome**
**Maebashi-shi Gunma-ken(JP)**

(74) Representative: **Clifford, Frederick Alan et al**
**MARKS & CLERK 57/60 Lincoln's Inn Fields**
**London WC2A 3LS(GB)**

## Description

This invention relates to a composition for periodontal use. More especially, it relates to a composition for periodontal use which can be applied for treatment and/or prevention of periodontal diseases, in particular alveolar pyorrhea.

The morbidity rate of periodontal disease is markedly increasing. According to dentists in general practice three in ten persons in the thirties already have the initial symptoms of alveolar pyorrhea. The initial symptoms begin with damage of keratine layer. Gums begin to bleed in biting (say) an apple or on tooth brushing, and bad breath is often a consequence. Healthy gingivae are usually pink-colored and stiff, whereas, in the initial stage of periodontal lesions, the gingivae deepen in color at the edges to a red coloration, sometimes dark red, increase in thickness with swelling, and bleed even on light touch. The exact mechnism of the progress of periodontal diseases is not known. It is assumed that proliferation of anaerobic bacteria occurs in the lesions and gives rise to alveolar pyorrhea. Alveolar pyorrhea then proceeds followed by pocket formation, loosening of teeth and finally loss of teeth. The loss rate of teeth due to such periodontal disease increases with age to exceed the loss rate by cariosity in the forties, and reach a maximum in the sixties. In other words, periodontal disease jeopardizes the tooth life, whereby it is a threat to general health in older people.

Current dietary habits of eating high proportions of cooked or processed food rather than raw or natural food, may affect, to a considerable degree, the morbidity rate of periodontal diseases. Prevention of periodontal disease has needed regular cleaning of dental plaque and periodontal elimination of dental calculus, together with stimulation and massage of gingivae. However, regular and strict execution of these techniques is not always easy in daily life.

Many other methods have been reported for prevention of periodontal diseases, for example, application of various kinds of antibacterial agents or of enzymes that decompose glucagon (which may be responsible for dental plaque formation) or use of electrolytes as an astringent for gingivae. However, none of these have been thoroughly successful. Although these expedients have also been tried for treatment of alveolar pyorrhea, they are only an auxiliary therapy to surgical operation. No drug effective against alveolar pyorrhea, has yet been found. For effective treatment of alveolar pyorrhea, surgical operations (such as scaling, pocket curettage, gingivectomy, flap operation or others) are currently necessary.

Nowadays, especially since a higher proportion of people survive to old age the development of a simple and non-surgical method for prevention and/or treatment of periodontal diseases, in particular alveolar pyorrhea is of major interest. The present invention sets out to provide an effective and simple method for prevention and/or treatment of periodontal diseases, in particular alveolar pyorrhea. The present invention further sets out to achieve prolongation of average tooth life.

I have found that, by applying superoxide dismutase to the lesion, periodontal diseases, in particular alveolar pyorrhea can be alleviated or in some instance completely cured and moreover that by applying superoxide dismutase to gingivae, periodontal diseases can be prevented.

Superperoxide dismutase isknown to be produced e.g. by cultivating a microorganism belonging to the Genus Serratia, and is known to be useful as in anti-inflammatory (EP 70656). Similarly, the publication 'La Recherche" Vol 10 No.106 Dec 1979, pages 1269 - 1270 discusses inter alia the anti-inflammatory properties and characteristics of superoxide dismutase. Once again no mention is made of any action specific to periodontal diseases.

Thus, the present invention consists in use of a composition containing superoxide dismutase for the manufacture of a medicament for the treatment of periodontal diseases

The process for manufacturing superoxide dismutase is known from earlier literature and patents. For example, it can be manufactured by extraction and purification from various organs such as liver, erythrocytes and placenta of animals (bovine or human) or from bacteria such as Escherichia coli, and genetically manipulated cells.

Superoxide dismutase has been proposed as a therapeutic agent for inflammatorical diseases (see EP 70656 above) especially osteoarthritis and rheumatoid arthritis; or (see La Recherche, above) cancer irradiation injury which are all possibly related to tissue damage due to superoxide. However, its half life in blood is as short as 6 minutes, and success in safe and effective treatment of human diseases has not been reported yet. There are no reports which suggest the application of superoxide dismutase to alveolar pyorrhea as in the present invention.

The superoxide dismutase to be employed for the purpose of the present invention may be any type of superoxide dismutase, and the invention is not limited by its source or manufacturing process. Unless the activity of superoxide dismutase is greatly impaired, any active portion of the enzyme, or a modified superoxide dismutase, can be used in the present invention and be included in the term 'superoxide dismutase' used in this specification. However, bovine or human superoxide dismutases are preferable.

The content of superoxide dismutase to be attained in the medicament depends on the form selected. Superoxide dismutase displays the effect of interest to the present invention even in small amount. The content in the made-up composition (medicament) will generally be in the range between about $1 \times 10^{-4}$ weight percent and about 1 weight percent based on the total weight of the composition (i.e. between about $3 \times 10^{-8}$ and about $3 \times 10^{-4}$ molar percent). When it is used for prevention, a lower concentration of superoxide dismutase can be employed.

When superoxide dismutase is applied to gingivae, it may be assumed to resolve superoxide to hydrogen peroxide. The latter may then react with chlorine ion naturally present to form hydrochlorite. Therefore, it is also advantageous to use catalase and/or one or more amino acids or derivatives thereof in the present composition in order to remove hydrogen peroxide and hypochlorite, respectively. For this purpose, commercially available catalase can be generally added in the concentration range between about $1 \times 10^{-4}$ and about 1 weight percent in the composition. Amino acids or derivatives thereof, preferably selected from taurine, glycine, aspartic acid, histidine, lysine and acyl glutamic acid (taurine is most preferable) can be present generally within the range from about 0.05 to about 5 weight percent of the total composition.

A composition for periodontal use, containing superoxide dismutase as an active ingredient, can be produced in any conventional form capable of application to gingivae.

The use of superoxide dismutase in treatment of periodontal diseases, in particular alveolar pyorrhea can for instance be manifested by injecting or infusing a solution into periodontal areas or pockets, or by flooding them in such solution. Thus, the medication made up by the present invention may be in a form of a solution for injection or infusion, e.g. such as a mouth wash. In order to keep superoxide dismutase in contact with gingivae for a sufficient time, the composition may alternatively be in the form of a tablet, (especially a tablet which on dissolution produces a liquid adhesive to gingivae), or a chewing-gum. It may alternatively be an oil-type or emulsion type ointment or gel formulation, which can be applied upon or rubbed into gingivae. A form especially suitable for prevention purposes is a conventional form of dentifrice such as a paste. Powder or semi-paste. For example, superoxide dismutase can be introduced into a conventional dentifrice.

Superoxide dismutase is stable against heat, has an optimum pH range of about 7 to 9 and a stable pH range of about 6 to 11. It is inactivated only in the presence of a strong acid or alkali, or a potent chelating agent. Thus, in manufacturing the present composition, any non-toxic conventional base and any conventional method well-known in the art can be employed. Moreover, in the present composition, other conventional active ingredients or adjuvant ingredients such as various kinds of enzymes, fluorine compounds, antibiotics, vitamins and other can also be added.

The present composition for injection or infusion can be manufactured by dissolving superoxide dismutase and optionally methylcellulose, sorbitol, serum albumin, preservatives and a flavoring agent, and after sterilisation, filling the solution into ampoules or vials. It may be in a lyophilized form.

Mouth wash can be produced by a conventional method employing optionally boric acid, borax and aluminium potassium sulfate. Flavoring agents, sweetening agents or preservatives can be added.

Tablets can be produced by any conventional method, employing the superoxide dismutase with optional ingredients chosen from diluting agents, binding agents, flavoring agents, coloring agents, lubricants, preservatives, sweetening agents and others. Examples of the diluting agents are various kinds of cellulose ethers, acrylate polymers, starch, dextrin, milk sugar, sorbitol and calcium phosphate. Examples of binding agents are starch, dextrin, gelatin, tragacanth and others. Further addition of polyvinyl alcohol can confer adhesiveness on the tablet.

Chewing-gum can also be produced by any conventional method, e.g. employing a gum base as vinyl acetate polymer, and binding agents, diluting agents, flavoring agents, coloring agents, preservatives and sweetening agents can be added as described above in connection with tablets.

Ointment or gel formulations can be produced employing a suitable base and additives depending on the properties and form desired. Examples of the bases are water, glycerol, 1,3-butanediol, proplylene glycol, polyethylene glycol, polypropylene glycol, ethanol, various kinds of cellulose ether, polyvinyl alcohol, carboxyvinyl alcohol, cetyl alcohol, vaseline and liquid paraffin. If necessary, tensides such as polyoxyethylene sorbitan fatty ester, polyoxyethylene fatter ester, polyoxyethylene alkyl ether and others can be combined in the preparation. Further, there may be added a flavoring agent, coloring agent, preservative and sweetening agent.

Dentifrices can be produced employing a suitable base and additives depending on the properties and form desired. Examples of the bases suitable for preparing paste, powder or semi-paste, are calcium phosphate, calcium carbonate, aluminium hydroxide, insoluble metaphosphoric acid, calcium pyrophosphate, magnesium carbonate, silicic acid and salts thereof and pulverised polymer. In addition, wetting agents such as glycerol, sorbitol, propylene glycol, polyethylene glycol and others, and binding agents such as bentonite, sodium carboxymethylcellulose, hydroxyvinyl polymer and tragacanth gum can be used. Futhermore, if necessary, tensides such as alkylsulfate, alklsulfonate, glycerol fatty acid ester, sorbitan fatty ester, flavoring agents, sweetening agents, coloring agents and preservatives can be added.

The present composition can be used at any stage of the periodontal disease for the purpose of treatment. The efficacies of the present composition and method are apparent from clinical studies using twenty patients with alveolar pyorrhea. After dental calculus was removed briefly with an ultrasonic scaler, a superoxide dismutase solution (Concentration : $0.5 \times 10^{-6}$ or $1.0 \times 10^{-6}$ M) was filled into the remaining dental pockets, which were then curetted slightly with a scaler. Next, the patients brushed their teeth and gingivae with a tooth-brush immersed in the above superoxide dismutase solution, and maintained for a while a pose which can keep the solution filled in the pockets. This therapy was repeated once a week for three weeks.

In this clinical study, all twenty cases showed improvement of alveolar pyorrhea. That is, the depth of pocket, as measured by a pocket probe, decreased from the average of 8.0 mm before treatment, to the average of 2.5 mm (range : 1 - 5 mm) after three times treatment (normal value : 1 - 2 mm). In most cases, violet or dark red colored gingivae were improved to pink-colored only one week after the first therapy. Subjective conditions such as pain and objective syndromes such as flail of teeth were also eliminated. No side effects were observed.

It is surprising that alveolar pyorrhea a disease, an effective conservation therapy for which has not hitherto been known was improved within a relatively short period, using only extremely small amounts of superoxide dismutase.

For the purpose of prevention, the use of superoxide dismutase in the form of usual dentifrices is preferable.

The present invention will be further illustrated in detail in the following examples without limiting the scope of the invention as claimed.

Example 1 : Solution for infusion

| Component | per 100 ml |
|---|---|
| Superoxide dismutase | 0.003 g |
| Methylcellulose | 3 g |
| Sorbitol | 10 g |
| Purified water | to 100 ml |

Methylcellulose is slowly added to purified water to give a homogeneous solution, to which is then added the rest of the components. After adjusting the total volume, the solution is filtered through a milipore filter for sterilisation, and filled into a suitable vial. A pale blue solution is obtained.

Example 2 : Solution for injection

A solution as described for infusion in Example 1 is made up and catalase (0.01 g/100 ml) and taurine (1 g/100 ml) is added.

Example 3 : Lyophilized preparation

Superoxide dismutase (0.01 g) is dissolved in 5% human serum albumin solution and the total volume is adjusted to 100 ml. Each 10 ml of the solution is filled into a 30 ml vial, and lyophilized. Prior to use, 10 ml of water is added to give a reproducible and infusible solution.

Example 4 : Tablet

| Component | (per tablet) |
|---|---|
| Superoxide dismutase | 0.001 mg |
| Microcrystalline cellulose | 120 mg |
| Magnesium stearate | 1.5 mg |
| Polyvinyl alcohol | 30 mg |
| Pectin | 9 mg |
| Hydrogenated oil | 3 mg |
| Milk sugar | 136.6 mg |
| Total | 300.0 mg |

The above components are mixed thoroughly and compressed to obtain a plain tablet. When dissolved in the mouth this tablet gives a liquid with adhesiveness to the gingivae, and can therefore release superoxide dismutase slowly.

Example 5 : Tablet

Tablets containing, in addition to the components as in the tablet of Example 4, catalase (0.01 mg/tablet) and taurine (10 mg/tablet), are prepared in the same manner as described in Example 4.

Example 6 : Oil-type ointment

| Component | | per 100 mg |
|---|---|---|
| A : | Superoxide dismutase | 0.003 g |
| | Purified water | 1.0 g |
| | Propylene glycol | 1.0 g |
| B : | Polyvinyl alcohol | 5.0 g |
| | Liquid paraffin | 40.0 g |
| | White vaselin | ad to 100 g |

The items of component B are combined and melted by heating to a temperature of 70 - 75° C on a water bath. After cooling of the melt to 45 - 50° C, the items of component A is added with stirring to produce a homogeneous oil type ointment.

Example 7 : Emulsion-type ointment

| Components | | per 100 g |
|---|---|---|
| A : | Superoxide dismutase | 0.01 g |
| B : | Stearyl alcohol | 5.0 g |
| | White vaseline | 8.0 g |
| | Liquid paraffin | 8.0 g |
| | Polyoxyethylene (20) sorbitan monostearate | 4.0 g |
| | Sorbitan monostearate | 2.0 g |
| | Glycerol fatty ester | 4.0 g |
| | Butyl p-hydroxybenzoate | 0.05 g |
| C : | Methyl p]hydroxybenzoate | 0.1 g |
| | Citric acid | 0.04 g |
| | Propylene gylcol | 10.0 g |
| | Purified water | to 100 g |

The items of component B are combined and melted by heating to a temperature of 70 - 75° C. The items of component C are then preheated to 70 - 75°C with stirring, and added to component B to obtain an emulsion. After stirring for 15 minutes, the emulsion is cooled to 50°C with water and the items of component A, dissolved in purified water, are added with stirring to produce a homogeneous emulsion-type ointment.

Example 9 : Aqueous gel

| Component | | |
|---|---|---|
| A : | Superoxide dismutase | 0.003 g |
| | Taurine | 1.0 g |
| B : | Carboxyvinyl polymer | 1.0 g |
| C : | Sodium hydroxide | q.s. |
| D : | Glycerol | 10.0 g |
| | Ethanol | 3.0 g |
| | Purified water | ad to total 100 g |

The mixture of items constituting D is slowly added to and dispersed homogeneously within component B. To this dispersion, the items of component A, dissolved in purified water, are added and homogeneously dissolved. The pH of the dispersion is adjusted to 6.5 with the sodium hydroxide (component C) to obtain an aqueous gel.

Example 10 : Tooth paste

| Component | | per 100 g |
|---|---|---|
| A : | Superoxide dismutase | 0.001 g |
| | Sodium lauryl sulfate | 2.0 g |
| | Sodium lauroyl sarcosinate | 0.2 g |
| | Calcium hydrogen phosphate | 40.0 g |
| | Magnesium phosphate | 0.5 g |
| | Silicic anhydride | 2.0 g |
| B : | Glycerol | 15.0 g |
| | Sorbitol | 10.0 g |
| | Sodium lactate | 2.0 g |
| | Carboxymethylcellurose | 1.0 g |
| | Methyl p-phydroxybenzoate | 0.05 g |
| | Ethyl p-hydroxybenzoate | 0.05 g |
| | Perfume | q.s |
| | Purfied water | ad to total 100 g |

The individual items of component B, except the perfume, are mixed into a homogeneous solution. This solution is combined with the items of component A successively under kneading. Finally the perfume is added. A homogeneous tooth paste is obtained.

**Claims**

1. Use of a composition containing superoxide dismutas for the manufacture of a medicament for the treatment of periodontal diseases .

2. Use of a composition according to Claim 1, characterised in that the superoxide dismutase is human superoxide dismutase.

3. Use of a composition according to Claim 1, characterised in that the superoxide dismutase is bovine superoxide dismutase.

4. Use of a composition according to any one of Claims 1 to 3, characterised in that in the medicament the superoxide dismutase is present in an amount within the range from $1 \times 10^{-4}$ weight percent to 1 weight percent based on the total weight of the composition.

5. Use of a composition according to any one of Claims 1 to 4 characterised in that the medicament further contains catalase.

6. Use of a composition according to Claim 5, characterised in that the catalase is present in the medicament in an amount within the range from $1 \times 10^{-4}$ weight percent to 1 weight percent based on the total weight thereof.

7. Use of a composition according to any one of Claims 1 to 6, characterised in that the medicament further contains one or more amino acids, or derivatives thereof.

8. Use of a composition acording to Claim 7, characterised in that the amino acid or derivative thereof in the medicament is selected from taurine, glycine, aspartic acid, histidine, lysine or acyl glutamic acid.

9. Use of a composition according to Claims 7 or 8, wherein the amino acid or derivative thereof is in the medicament in an amount within the range from 0.05 weight percent to 5 weight percent based on the total weight of the composition.

10. Use of a composition according to any one of the preceeding claims, characterised in that the medicament is presented in the form of solution, a solid composition, an ointment or gel or a dentifrice.

11. Use of a composition according to any one of Claims 1 to 9 characterised in that the medicament is presented in the form of an injectible solution, an infusible solution, a mouthwash or oral lotion, a tablet, a chewable tablet forming a liquid adherent to gingivae, a chewing-gum, on oil-type ointment, an emulsion-type ointment, a paste dentifrice, a powder dentifrice or a semi-paste dentifrice.

## Revendications

1. Utilisation d'une composition contenant de la superoxyde dismutase pour la préparation d'un médicament pour le traitement des parodontolyses.

2. Utilisation d'une composition selon la revendication 1, caractérisée en ce que la superoxyde dismutase est de la superoxyde dismutase humaine.

3. Utilisation d'une composition selon la revendication 1, caractérisée en ce que la superoxyde dismutase est une superoxyde dismutase de bovins.

4. Utilisation d'une composition selon l'une quelconque des revendications 1 à 3, caractérisée en ce que dans le médicament, la superoxyde dismutase est présente dans une quantité dans l'intervalle de $1 \times 10^{-4}$ % en poids à 1 % en poids par rapport au poids total de la composition.

5. Utilisation d'une composition selon l'une quelconque des revendications 1 à 4, caractérisée en ce que le médicament contient en outre de la catalase.

6. Utilisation d'une composition selon la revendication 5, caractérisée en ce que la catalase est présente dans le médicament dans une quantité dans l'intervalle de $1 \times 10^{-4}$ % en poids à 1 % en poids par rapport au poids total de celui-ci.

7. Utilisation d'une composition selon l'une quelconque des revendications 1 à 6, caractérisée en ce que le médicament contient en outre un ou plusieurs amino-acides, ou dérivés de ceux-ci.

8. Utilisation d'une composition selon la revendication 7, caractérisée en ce que l'amino-acide ou son dérivé présent dans le médicament est choisi parmi la taurine, la glycine, l'acide aspartique, l'histidine,

7

la lysine ou l'acide acyl glutamique.

9. Utilisation d'une composition selon les revendications 7 ou 8, dans laquelle l'amino-acide ou son dérivé est présent dans le médicament dans une quantité dans l'intervalle de 0,05 % en poids à 5 % en poids par rapport au poids total de la composition.

10. Utilisation d'une composition selon l'une quelconque des revendications précédentes, caractérisée en ce que le médicament est présenté sous la forme d'une solution, d'une composition solide, d'une pommade ou d'un gel ou d'un dentifrice.

11. Utilisation d'une composition selon l'une quelconque des revendications 1 à 9, caractérisée en ce que le médicament est présenté sous la forme d'une solution injectable, d'une solution perfusable, d'un bain de bouche ou d'une lotion buccale, d'un comprimé, d'un comprimé à mâcher formant un liquide adhérant aux gencives, d'une gomme à mâcher, d'une pommade du type huile, d'une pommade du type émulsion, d'une pâte dentifrice, d'une poudre dentifrice ou d'un dentifrice semi-pâteux.

**Patentansprüche**

1. Verwendung einer Zusammensetzung enthaltend Superoxid-Dismutase für die Herstellung eines Heilmittels zur Behandlung von periodontalen Erkrankungen.

2. Verwendung einer Zusammensetzung nach Anspruch 1, dadurch gekennzeichnet, daß die Superoxid-Dismutase humane Superoxid-Dismutase ist.

3. Verwendung einer Zusammensetzung nach Anspruch 1, dadurch gekennzeichnet, daß die Superoxid-Dismutase Rinder-Superoxid-Dismutase ist.

4. Verwendung einer Zusammensetzung nach einem der Ansprüche 1 bis 3, dadurch gekennzeichnet, daß die Superoxid-Dismutase in einer Menge von $1 \times 10^{-4}$ bis 1 Gew.-% bezogen auf das gesamte Gewicht der Zusammensetzung in dem Pharmazeutikum vorliegt.

5. Verwendung einer Zusammensetzung nach einem der Ansprüche 1 bis 4, dadurch gekennzeichnet, daß das Pharmazeutikum zusätzlich Katalase enthält.

6. Verwendung einer Zusammensetzung nach Anspruch 5, dadurch gekennzeichnet, daß die Katalase in einer Menge von $1 \times 10^{-4}$ bis 1 Gew.-% bezogen auf das gesamte Gewicht des Pharmazeutikums in diesem vorliegt.

7. Verwendung einer Zusammensetzung nach einem der Ansprüche 1 bis 6, dadurch gekennzeichnet, daß das Pharmazeutikum weiters eine oder mehrere Aminosäure(n) oder Derivate derselben enthält.

8. Verwendung einer Zusammensetzung nach Anspruch 7, dadurch gekennzeichnet, daß die Aminosäure oder deren Derivat Taurin, Glycin, Aspartinsäure, Histidin, Lysin und Acylglutaminsäure ist.

9. Verwendung einer Zusammensetzung nach Anspruch 7 oder 8, dadurch gekennzeichnet, daß die Aminosäure oder deren Derivat in einer Menge von 0,05 bis 5 Gew.-% bezogen auf das gesamte Gewicht der Zusammensetzung im Pharmazeutikum vorliegt.

10. Verwendung einer Zusammensetzung nach einem der Ansprüche 1 bis 9, dadurch gekennzeichnet, daß das Pharmazeutikum in Form einer Lösung, Feststoffzusammensetzung, Salbe, Gel oder Zahnpaste dargereicht ist.

11. Verwendung einer Zusammensetzung nach einem der Ansprüche 1 bis 9, dadurch gekennzeichnet, daß das Pharmazeutikum in Form einer injizierbaren Lösung, einer Infusion, eines Mundspül- oder -waschmittels, einer Tablette, einer Kautablette zur Ausbildung eines flüssigen Haftmittels an der Gingiva, eines Kaugummis, einer Salbe vom Öl-Typ, einer Salbe vom Emulsionstyp, einer Zahnpaste, eines Zahnputzpulvers oder eines semi-pastösen Zahnpflegemittels vorliegt.